# EUROPEAN PATENT APPLICATION

(11) **EP 4 300 083 A1**
(43) Date of publication of application: **03.01.2024**
(21) Application number: 22758752.4
(22) Date of filing: 28.01.2022
(51) Int. Cl.: G01N 23/046

(54) **IMAGING SYSTEM FOR RADIOGRAPHIC EXAMINATION**

(30) Priority: 26.02.2021 CN 202110217737
(71) Applicant: Tsinghua University, Beijing 100085 (CN); Nuctech Company Limited, TongFang Building Shuangqinglu Haidian District Beijing 100084 (CN)
(72) Inventor: CHEN, Zhiqiang, Beijing 100084 (CN); ZHANG, Li, Beijing 100084 (CN); JIN, Xin, Beijing 100084 (CN); ZHAO, Zhenhua, Beijing 100084 (CN)
(74) Representative: Cabinet Beau de Loménie
(86) International application number: PCT/CN2022/074837
(87) International publication number: WO 2022/179387

(57) **Abstract**

An imaging system for radiographic examination, the imaging system comprising: a first radiation source assembly (10) comprising a plurality of radiation sources (100); a second radiation source assembly (20) comprising a plurality of radiation sources (100), with target points of all the radiation sources (100) of the first radiation source assembly (10) being arranged in a first radiation source plane, and target points of all the radiation sources (100) of the second radiation source assembly (20) being arranged in a second radiation source plane; a plurality of first detector units (310), the plurality of first detector units (310) being arranged in a first detector plane; a plurality of second detector units (320), the plurality of second detector units (320) being arranged in a second detector plane; and a detector holder (330), the plurality of first detector units (310) and the plurality of second detector units (320) being all mounted on the detector holder (330), wherein the first radiation source plane, the second radiation source plane, the first detector plane and the second detector plane are sequentially distributed in an advancing direction.

## Description

### CROSS REFERENCES TO RELATED APPLICATIONS

The present disclosure claims the priority to the Chinese patent application No. 202110217737.9, filed on February 26, 2021 and titled by "IMAGING SYSTEM FOR RADIOGRAPHIC INSPECTION", which is incorporated here by reference in its entirety.

### TECHNICAL FIELD

The present disclosure relates to a technical field of fluoroscopy imaging technology, in particular to an imaging system for radiographic inspection.

### BACKGROUND

Distributed ray sources have been widely used in the field of fluoroscopy imaging, for example, in CT (computed tomography) device for object inspection, medical diagnosis, and the like. CT scanning generates three-dimensional scanning images and has high recognition ability. According to motion of a ray source relative to an object under inspection during a scanning process, the existing CT scanning system can include a dynamic spiral CT scanning system and a static CT scanning system.

In the dynamic spiral CT scanning system, the ray source continuously rotates around the object under inspection during the scanning process, while the conveying device uniformly and horizontally transports the object under inspection through an inspection area. The dynamic spiral CT scanning system typically requires a slip ring and a bearing, and during the scanning process, the slip ring needs to rotate at high speed. In the existing static CT scanning system, the ray source remains stationary throughout the entire scanning process, and an integrated ray source disposed around the inspection area is adopted for scanning.

Compared to the dynamic spiral CT using the slip ring, the static CT scanning system can have a more flexible design. For example, the static CT scanning system can have different scanning channel sizes, device heights, and transport speeds according to application requirements. Since the slip ring is not required, the static CT scanning system can reduce noise and cost, and can achieve instant scanning and stopping, thereby shortening the preparation time.

According to CT reconstruction theory, only when a certain scanning angle (for example, at least 120 degrees) is achieved for the scanned object in the CT scanning system, completeness of data can be met and accuracy of numerical reconstruction can be ensured. One single distributed ray source is generally unable to meet such requirement due to limitations of mechanical structures. When multiple distributed ray sources are combined and arranged, it is difficult to meet the data completeness requirements by disposing the multiple distributed ray sources in the same plane, due to structural interferences between different ray sources or between the ray sources and detectors. Generally, multiple (three or more) planes are needed, where one ray source group and one corresponding detector group are arranged on each plane, and a beam surface of the ray source coincides with a central plane of the detector. Such arrangement increases the size of the entire system and the number of detectors.

Therefore, an improved imaging system and method are needed.

### SUMMARY

One object of the present disclosure is to provide an imaging system which can avoid structural interferences. One object of the present disclosure is to provide an imaging system which can meet the requirements of CT reconstruction. One object of the present disclosure is to provide an imaging system which can improve the imaging quality. One object of the present disclosure is to provide an imaging system which can reduce sizes and costs of devices. One object of the present disclosure is to provide an imaging system in which optical paths can be designed flexibly.

One aspect of the present disclosure provides an imaging system for radiographic inspection, including: an inspection area, wherein an object under inspection can be transported through the inspection area in a direction of travel; a first ray source assembly, including a plurality of ray sources; a second ray source assembly, including a plurality of ray sources, wherein each ray source of the first ray source assembly and of the second ray source assembly includes a separate shell to define a vacuum space and includes a plurality of target spots enclosed in the shell, the target spots of all the ray sources of the first ray source assembly are arranged in a first ray source plane, and the target spots of all the ray sources of the second ray source assembly are arranged in a second ray source plane; a plurality of first detector units, adapted for receiving X-rays that are emitted from the first ray source assembly and pass through the inspection area, the plurality of first detector units being arranged in a first detector plane; a plurality of second detector units, adapted for receiving X-rays that are emitted from the second ray source assembly and pass through the inspection area, the plurality of second detector units being arranged in a second detector plane; and a detector bracket, wherein the plurality of first detector units and the plurality of second detector units are both installed on the detector bracket, wherein the first ray source plane, the first detector plane, the second detector plane, and the second ray source plane are distributed in sequence in the direction of travel.

According to some embodiments of the present disclosure, the first ray source plane, the first detector plane, the second detector plane, and the second ray source plane are substantially perpendicular to the direction of travel and parallel to each other.

According to some embodiments of the present disclosure, the imaging system is further configured to reconstruct a three-dimensional scanning image of the object under inspection based on detection data from the plurality of first detector units and the plurality of second detector units.

According to some embodiments of the present disclosure, the imaging system further includes a ray source control device for controlling ray emission of the first ray source assembly and the second ray source assembly, wherein the ray source control device is configured so that at the same moment, at most one target spot in the first ray source assembly emits X-rays and at most one target spot in the second ray source assembly emits X-rays.

According to some embodiments of the present disclosure, the plurality of first detector units cover at least a ray emission range of the first ray source assembly, and the plurality of second detector units cover at least a ray emission range of the second ray source assembly.

According to some embodiments of the present disclosure, the plurality of first detector units are configured to extend completely around the inspection area to form a first detector ring; and/or the plurality of second detector units are configured to extend completely around the inspection area to form a second detector ring.

According to some embodiments of the present disclosure, when observed in the direction of travel, distribution positions of the target spots in the first ray source assembly do not completely coincide with distribution positions of the target spots in the second ray source assembly.

According to some embodiments of the present disclosure, when observed in the direction of travel, the distribution positions of the target spots in the first ray source assembly are staggered from those of the target spots in the second ray source assembly.

According to some embodiments of the present disclosure, when observed in the direction of travel, a projection of each ray source of the first ray source assembly does not completely fall within a projection of any ray source of the second ray source assembly, and the projection of each ray source of the second ray source assembly does not completely fall within the projection of any ray source of the first ray source assembly.

According to some embodiments of the present disclosure, when observed in the direction of travel, projections of the plurality of ray sources of the first ray source assembly are staggered from those of the plurality of ray sources of the second ray source assembly.

According to some embodiments of the present disclosure, all the target spots of each ray source of the first ray source assembly are arranged to deflect towards the plurality of first detector units in the direction of travel by a first deflection angle, so that the X-rays emitted by each ray source of the first ray source assembly will not be blocked by the plurality of first detector units before passing through the inspection area; and/or all the target spots of each ray source of the second ray source assembly are arranged to deflect towards the plurality of second detector units in the direction of travel by a second deflection angle, so that the X-rays emitted by each ray source of the second ray source assembly will not be blocked by the plurality of second detector units before passing through the inspection area.

According to some embodiments of the present disclosure, the first ray source assembly further includes a first collimator adapted for deflecting a direction of the X-rays emitted by the first ray source assembly towards the plurality of first detector units in the direction of travel by a first deflection angle; and/or the second ray source assembly further includes a second collimator adapted for deflecting a direction of the X-rays emitted by the second ray source assembly towards the plurality of second detector units in the direction of travel by a second deflection angle.

According to some embodiments of the present disclosure, when observed in the direction of travel, the first ray source assembly and the second ray source assembly have a combined scanning angle greater than 120 degrees relative to the inspection area.

According to some embodiments of the present disclosure, when observed in the direction of travel, the first ray source assembly and the second ray source assembly have a combined scanning angle greater than 180 degrees relative to the inspection area.

According to some embodiments of the present disclosure, at least one target spot in the first ray source assembly and the second ray source assembly is a digital radiographic (DR) target spot, and the ray source control device is configured so that a ray emission frequency of the digital radiographic target spot is higher than that of other target spots.

According to some embodiments of the present disclosure, the first detector unit includes a single row of detector crystals or multiple rows of detector crystals, and/or the second detector unit includes a single row of detector crystals or multiple rows of detector crystals.

According to some embodiments of the present disclosure, the imaging system includes two ray source assemblies and one detector assembly, the two ray source assemblies form two ray source planes, respectively located on two sides of the detector assembly, and the detector assembly includes two groups of detector units, respectively corresponding to the two ray source assemblies and forming two detector planes. In some embodiments, two groups of detector units are installed on the same detector bracket, which can effectively shorten the length and weight of the imaging device and reduce the difficulty of radiation protection. In some embodiments, the ray sources in each ray source assembly are arranged without interference with each other, and each ray source plane is spaced apart from the corresponding detector plane. Therefore, the imaging system can avoid interferences between the ray sources and that between the ray sources and the detectors, resulting in a larger scanning angle covering the inspection area and providing more complete detection data. In some embodiments, each ray source assembly includes a plurality of ray sources, and the detectors in the corresponding groups of detector units can be shared by the plurality of ray sources, thereby reducing the cost of the imaging system. In some embodiments, the distribution positions of target spots of the two ray source assemblies can complement each other, thereby obtaining more complete projection data and improving imaging quality and accuracy. In some embodiments, the arrangements of target spots of the ray sources and detectors in the imaging system, as well as the design of optical paths, are more flexible and can meet more application requirements.

### BRIEF DESCRIPTION OF THE DRAWINGS

Fig. 1 is a schematic diagram of an imaging system according to some embodiments of the present disclosure.
Fig. 2 is a perspective view of an imaging system according to some embodiments of the present disclosure.
Fig. 3 is a schematic diagram of a detector assembly according to some embodiments of the present disclosure.
Fig. 4 is a schematic diagram showing a relative position of a ray source assembly and a detector assembly according to some embodiments of the present disclosure.
Fig. 5 is a schematic diagram of an imaging system according to some embodiments of the present disclosure.
Fig. 6A is a cross-sectional schematic diagram of a ray source and a detector unit according to some embodiments of the present disclosure.
Fig. 6B is a cross-sectional schematic diagram of a ray source and a detector unit according to some embodiments of the present disclosure.
Fig. 7 is a schematic diagram showing distribution of target spots in an imaging system according to some embodiments of the present disclosure.

### DETAILED DESCRIPTION

Below, embodiments of the present disclosure are described with reference to the accompanying drawings. The following detailed description and accompanying drawings are used to illustrate the principle of the present disclosure by examples. The present disclosure is not limited to the preferred embodiments as described, and the scope of the present disclosure is defined by the claims. The present disclosure is now described in detail with reference to exemplary implementations, some of which are illustrated in the accompanying drawings. The following description is made with reference to the accompanying drawings, and unless otherwise indicated, the same reference numerals in different drawings represent the same or similar elements. The solutions described in the following exemplary embodiments do not represent all the solutions of the present disclosure. On the contrary, these solutions are only examples of systems and methods of various aspects of the present disclosure involved in the appended claims.

The imaging system for radiographic inspection according to the embodiments of the present disclosure can be applied to an inspection system for performing fluoroscopic imaging inspection of objects or human bodies.

According to some embodiments of the present disclosure, the inspection system may include an imaging system and a conveying device. The conveying device is adapted to transport an objection under inspection through an inspection area in a direction of travel. In an exemplary embodiment, the direction of travel is substantially parallel to a horizontal direction. In some embodiments, the conveying device transports the object under inspection in a uniform linear motion.

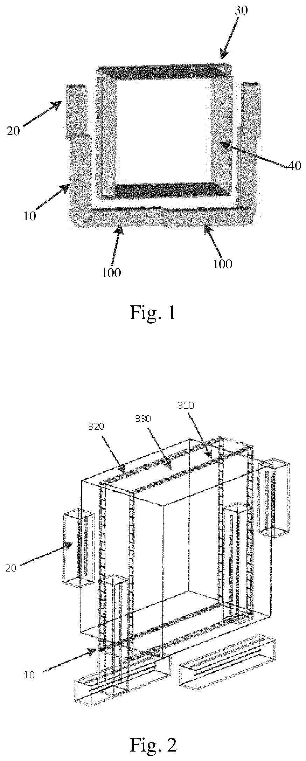

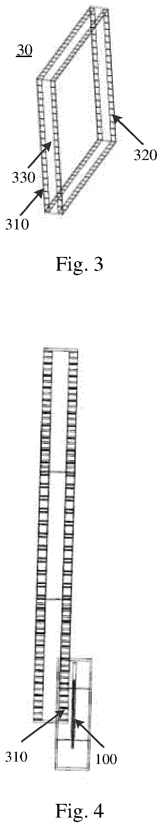

Fig. 1 is a schematic diagram of an imaging system according to some embodiments of the present disclosure. Fig. 2 is a perspective view of an imaging system according to some embodiments of the present disclosure. Fig. 3 is a schematic diagram of a detector assembly according to some embodiments of the present disclosure. Fig. 4 is a schematic diagram showing a relative position of a ray source assembly and a detector assembly according to some embodiments of the present disclosure.

According to some embodiments of the present disclosure, as shown in Fig. 1, the imaging system includes two ray source assemblies 10, 20, and a detector assembly 30. The ray source assemblies 10, 20 are adapted to emit X-rays. The detector assembly 30 is adapted to receive X-rays that are emitted from the ray source assemblies 10, 20 and pass through the inspection area. According to some embodiments of the present disclosure, the ray source assemblies 10, 20, and the detector assembly 30 remain stationary during operation of the imaging system, that is, the imaging system is a static imaging system.

According to some embodiments of the present disclosure, the imaging system defines an inspection area. In this article, "inspection area" means that the object under inspection can be transported through the inspection area in the direction of travel. When the object under inspection is located in the inspection area, the X-rays emitted from the ray source assemblies 10, 20 can penetrate the object under inspection and be received by the detector assembly 30. In an exemplary embodiment, the inspection area includes a first end and a second end. In some embodiments, the object under inspection is transported into the inspection area from one of the first and second ends, and is transported out of the inspection area from the other end.

According to some embodiments of the present disclosure, as shown in Fig. 1, the imaging system may further include a shielding component 40. In an exemplary embodiment, the shielding component 40 may be disposed around the inspection area of the imaging system. In an exemplary embodiment, the ray source assemblies 10, 20, and the detector assembly 30 are disposed on an outer side of the inspection area, that is, on a side of the shieling component 40 far away from the inspection area.

The structure of the ray source assembly according to some embodiments of the present disclosure is described in detail below with reference to the accompanying drawings.

According to some embodiments of the present disclosure, the ray source assembly 10 includes a plurality of ray sources 100. According to some embodiments of the present disclosure, the ray source assembly 20 includes a plurality of ray sources 100. In some embodiments shown in Figs. 1 and 2, the ray source assemblies 10 and 20 each include three ray sources 100.

The ray source assembly 10 and ray source assembly 20 described above respectively include a plurality of ray sources 100. However, the present disclosure is not limited to this. According to some embodiments of the present disclosure, the ray source assembly 10 or the ray source assembly 20 may include one or more ray sources 100.

According to some embodiments of the present disclosure, each ray source 100 is a distributed ray source. In an exemplary embodiment, each ray source 100 includes a separate shell to define a separate vacuum space. Each ray source 100 includes a plurality of target spots (not shown in Figs. 1 and 2) enclosed in the shell. In an exemplary embodiment, the plurality of target spots of each ray source 100 have uniform target spot spacing.

As mentioned above, when the imaging system includes a plurality of ray sources 100, each ray source 100 defines a separate vacuum space and therefore does not share the vacuum space with other ray sources 100. The vacuum spaces of different ray sources 100 are not communicated with each other. According to some embodiments of the present disclosure, each ray source 100 can be detached from and/or installed into the imaging system independently of other ray sources 100.

In some embodiments, the plurality of target spots of each ray source 100 can be distributed in a straight line. In some embodiments, the shell of the ray source 100 can correspond to the distribution of the target spots in shape. For example, when the target spots of the ray source 100 are distributed in the straight line, the shell of ray source 100 can have a linear outer contour, as shown in Fig. 1.

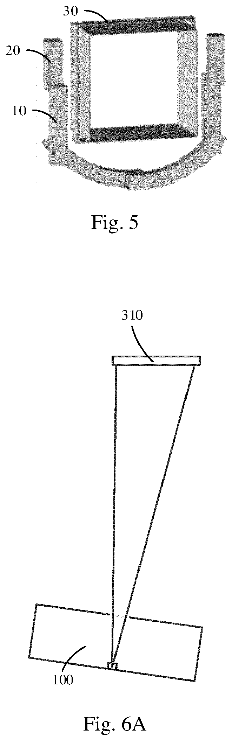

As described above, the plurality of target spots in each ray source 100 are distributed in a straight line. However, the present disclosure is not limited to this. In some embodiments, the plurality of target spots in the ray source 100 can further be distributed in an arc line, a polyline, or the like. Fig. 5 is a schematic diagram of an imaging system according to some embodiments of the present disclosure. In some embodiments, as shown in Fig. 5, the plurality of target spots (not shown in Fig. 5) in the ray source 100 located below the inspection area are distributed in an arc line. Correspondingly, when observed in the direction of travel, the shell of the ray source 100 may have an arc-shaped outer contour. In this case, with the arc-shaped contour, the ray source 100 can provide sufficient space for installation of the conveying device.

According to some embodiments of the present disclosure, the plurality of ray sources 100 of the ray source assembly 10 may have the same or different sizes. According to some embodiments of the present disclosure, the plurality of ray sources 100 of the ray source assembly 20 may have the same or different sizes. For example, as shown in Figs. 1 and 2, the three ray sources of the ray source assembly 10 have two different sizes, and the three ray sources of the ray source assembly 20 also have two different sizes.

According to some embodiments of the present disclosure, each ray source 100 of the ray source assemblies 10 and 20 has a ray emission range. According to some embodiments of the present disclosure, when the ray source assembly 10 includes a plurality of ray sources 100, the plurality of ray sources 100 of the ray source assembly 10 can provide a combined ray emission range. In some embodiments, the combined ray emission range of the ray source assembly 10 may be continuous or discontinuous. According to some embodiments of the present disclosure, when the ray source assembly 20 includes a plurality of ray sources 100, the plurality of ray sources 100 of the ray source assembly 20 can provide a combined ray emission range. In some embodiments, the combined ray emission range of the ray source assembly 20 may be continuous or discontinuous.

In some embodiments, the ray emission ranges of the ray source assemblies 10 and 20 are selected so that the entire object under inspection can fall within the ray emission ranges. As a result, the object under inspection can undergo a complete fluoroscopic inspection, thereby improving an imaging integrity of the imaging system.

In some embodiments, the ray emission ranges of the ray source assemblies 10 and 20 are selected so that a part of interest of the object under inspection can fall within the ray emission ranges. As a result, it is possible for the fluoroscopic inspection to focus merely on the part of interest of the object under inspection, thereby reducing the power consumption and cost of the imaging system while meeting imaging requirements.

According to some embodiments of the present disclosure, when the ray source assembly 10 includes a plurality of ray sources 100, the target spots of all the ray sources 100 of the ray source assembly 10 are arranged in the same plane (hereinafter referred to as "a first ray source plane"), that is, the plurality of ray sources 100 of the ray source assembly 10 are coplanar. According to some embodiments of the present disclosure, when the ray source assembly 20 includes a plurality of ray sources 100, the target spots of all the ray sources 100 of the ray source assembly 20 are arranged in the same plane (hereinafter referred to as "a second ray source plane"), that is, the plurality of ray sources 100 of the ray source assembly 20 are coplanar. In this article, "the target spots ...... are arranged in the same plane" means that beam exiting points of the target spots are all arranged in the same plane.

In an exemplary embodiment, the first ray source plane is substantially parallel to the second ray source plane. In some embodiments, the first ray source plane is substantially perpendicular to the direction of travel. In some embodiments, the second ray source plane is substantially perpendicular to the direction of travel.

According to some embodiments of the present disclosure, the plurality of ray sources 100 of the ray source assembly 10 are arranged without interference with each other. In some embodiments, when the ray source assembly 10 includes a plurality of ray sources 100, the plurality of ray sources 100 of the ray source assembly 10 are spaced apart from each other around the inspection area. In some embodiments, when the ray source assembly 10 includes a plurality of ray sources 100, the plurality of ray sources 100 of the ray source assembly 10 are distributed continuously around the inspection area. In some embodiments, for example, as shown in Fig. 1, when observed in the direction of travel, the plurality of ray sources 100 of the ray source assembly 10 may include a ray source 100 located below the inspection area, a ray source 100 located on a left side of the inspection area, and/or a ray source 100 located on a right side of the inspection area.

According to some embodiments of the present disclosure, the plurality of ray sources 100 of the ray source assembly 20 are arranged without interference with each other. In some embodiments, when the ray source assembly 20 includes a plurality of ray sources 100, the plurality of ray sources 100 of the ray source assembly 20 are spaced apart from each other around the inspection area. In some embodiments, when the ray source assembly 20 includes a plurality of ray sources 100, the plurality of ray sources 100 of the ray source assembly 20 are distributed continuously around the inspection area. In some embodiments, for example, as shown in Fig. 1, when observed in the direction of travel, the plurality of ray sources 100 of the ray source assembly 20 may include a ray source 100 located below the inspection area, a ray source 100 located on a left side of the inspection area, and/or a ray source 100 located on a right side of the inspection area.

The structure of a detector assembly according to some embodiments of the present disclosure is described in detail below with reference to the accompanying drawings.

According to some embodiments of the present disclosure, for example, as shown in Fig. 3, the detector assembly 30 includes a plurality of first detector units 310, a plurality of second detector units 320, and a detector bracket 330. The plurality of first detector units 310 are adapted to receive X-rays that are emitted from the ray source assembly 10 and pass through the inspection area. The plurality of second detector units 320 are adapted to receive X-rays that are emitted from the ray source assembly 20 and pass through the inspection area. According to some embodiments of the present disclosure, the plurality of first detector units 310 and the plurality of second detector units 320 are both installed on the detector bracket 330.

According to some embodiments of the present disclosure, each detector unit 310, 320 may be a single energy detector unit, a dual energy detector unit, an energy spectrum detector unit, or the like. However, it should be understood that the type of the detector unit in the present disclosure is not limited to the three types as mentioned above.

In an exemplary embodiment, the plurality of first detector units 310 are arranged in the same plane (hereinafter referred to as "a first detector plane"). In an exemplary embodiment, the plurality of second detector units 320 are arranged in the same plane (hereinafter referred to as the "a second detector plane"). In this article, "the detector units are arranged in the same plane" means that central planes of the detector units (such as central planes of detector crystals) are all arranged in the same plane. For example, a same positioning reference may be adopted so that the central planes of the plurality of first detector units 310 or of the plurality of second detector units 320 are arranged in the same plane. In some embodiments, the first detector unit 310 includes a single row of detector crystals or multiple rows of detector crystals, and/or the second detector unit 320 includes a single row of detector crystals or multiple rows of detector crystals.

In an exemplary embodiment, the first detector plane is substantially parallel to the second detector plane. In some embodiments, the first detector plane is substantially perpendicular to the direction of travel. In some embodiments, the second detector plane is substantially perpendicular to the direction of travel.

In some embodiments, the first ray source plane of the ray source assembly 10 is spaced apart from the first detector plane of the plurality of first detector units 310 in the direction of travel. In an exemplary embodiment, the first ray source plane of the ray source assembly 10 is substantially parallel to the first detector plane of the plurality of first detector units 310.

In some embodiments, the second ray source plane of the ray source assembly 20 is spaced apart from the second detector plane of the plurality of second detector units 320 in the direction of travel. In an exemplary embodiment, the second ray source plane of the ray source assembly 20 is substantially parallel to the second detector plane of the plurality of second detector units 320.

According to some embodiments of the present disclosure, the first ray source plane, the first detector plane, the second detector plane, and the second ray source plane are distributed in sequence in the direction of travel. In an exemplary embodiment, for example as shown in Fig. 2, the ray source assembly 10 and the ray source assembly 20 are respectively disposed on two sides of the detector assembly 30 in the direction of travel. As a result, the imaging system according to some embodiments of the present disclosure includes two ray source planes respectively located on two sides of the detector assembly.

In some embodiments, the first ray source plane, the first detector plane, the second detector plane, and the second ray source plane are substantially parallel to each other. In some embodiments, the first ray source plane, the first detector plane, the second detector plane, and the second ray source plane are substantially perpendicular to the direction of travel.

In some embodiments, the detector bracket 330 includes a detector bracket central plane, for example as shown in Fig. 2. In this article, the "detector bracket central plane" represents the geometric central plane of the detector bracket 330 in the direction of travel. According to some embodiments of the present disclosure, the detector bracket central plane is substantially parallel to the first ray source plane and/or the second ray source plane. In some embodiments, the detector bracket central plane is substantially parallel to the first detector plane and/or the second detector plane. In an exemplary embodiment, the detector bracket central plane is substantially perpendicular to the direction of travel. According to some embodiments of the present disclosure, the first ray source plane, the detector bracket central plane, and the second ray source plane are distributed in sequence in the direction of travel. Therefore, the imaging system according to some embodiments of the present disclosure includes two ray source planes and one detector bracket central plane, and the two ray source planes are located on two sides of the detector bracket central plane.

According to some embodiments of the present disclosure, the ray source assemblies 10, 20, and the detector assembly 30 are arranged without interference with each other. In some embodiments, in the direction of travel, the plurality of ray sources 100 of the ray source assembly 10 are spaced apart from adjacent outermost part of the detector assembly 30 (such as the outermost part of the plurality of first detector units 310 or the detector bracket 330 opposite the ray source assembly 10) at a predetermined distance. In some embodiments, in the direction of travel, the plurality of ray sources 100 of the ray source assembly 20 are spaced apart from adjacent outermost part of the detector assembly 30 (for example, the outermost part of the plurality of second detector units 320 or the detector bracket 330 opposite the ray source assembly 20) at a predetermined distance. In some embodiments, as shown in Fig. 4, the detector units 310 (or 320) are spaced part from the target spots of the ray sources 100. As such, the X-rays emitted by the ray source assembly 10 or 20 will not be blocked by the detector assembly 30 before passing through the inspection area.

In some embodiments, an arrangement of detector units in the detector assembly 30 can be provided according to factors such as arrangements of the ray source assemblies 10 and 20, and/or a size of the object under inspection. In some embodiments, the arrangement of detector units in the detector assembly 30 can further adopt a cost-effective arrangement, that is, meet the imaging requirements with detector units as few as possible.

According to some embodiments of the present disclosure, the plurality of first detector units 310 of the detector assembly 30 are arranged to cover at least the ray emission range of the ray source assembly 10. According to some embodiments of the present disclosure, the plurality of second detector units 320 of the detector assembly 30 are arranged to cover at least the ray emission range of the ray source assembly 20. As a result, the detector assembly 30 can cover the ray emission ranges of the ray source assemblies 10 and 20, and thus can cover the entire imaging range in the inspection area. In this case, the arrangement of the detector assembly 30 can fully utilize the X-rays emitted by the ray sources, and the imaging quality and inspection accuracy can be improved.

In some embodiments, the plurality of first detector units 310 are arranged to cover at least a portion of the ray emission range of the ray source assembly 10 (for example, the ray emission range corresponding to the part of interest of the objection under inspection). According to some embodiments of the present disclosure, the plurality of second detector units 320 are arranged to cover at least a portion of the ray emission range of the ray source assembly 20 (for example, the ray emission range corresponding to the part of interest of the objection under inspection). As a result, the detector assembly 30 can cover the selected ray emission ranges of the ray source assemblies 10 and 20, and thus can cover the main imaging range in the inspection area. In this case, the arrangement of the detector assembly 30 can reduce the cost of the imaging system while ensuring sufficient imaging quality and inspection accuracy.

In an exemplary embodiment, the plurality of first detector units 310 of the detector assembly 30 extend completely around the inspection area. Therefore, the plurality of first detector units 310 form a complete and continuous first detector ring. In some embodiments, the first detector ring may be a circular ring, a square ring, a rectangular ring, a polygonal ring, or the like. For example, as shown in Figs. 2 and 3, the plurality of first detector units 310 form a square ring.

In an exemplary embodiment, the plurality of second detector units 320 of the detector assembly 30 extend completely around the inspection area. Therefore, the plurality of second detector units 320 form a complete and continuous second detector ring. In some embodiments, the second detector ring may be a circular ring, a square ring, a rectangular ring, a polygonal ring, or the like. For example, as shown in Figs. 2 and 3, the plurality of second detector units 320 form a square ring.

As described above, the plurality of first detector units 310 or the plurality of second detector units 320 form a complete detector ring. However, the present disclosure is not limited to this. In some embodiments, the detector ring composed of the plurality of first detector units 310 or the plurality of second detector units 320 may be incomplete, i.e. there may be gaps in the ring. In some embodiments, the plurality of first detector units 310 are divided into multiple segments, and the first detector units 310 of different segments can be spaced apart from each other around the inspection area. In some embodiments, the plurality of second detector units 320 are divided into multiple segments, and the second detector units 320 of different segments can be spaced apart from each other around the inspection area.

According to some embodiments of the present disclosure, the imaging system can be configured to reconstruct a three-dimensional scanning (CT) image of the objection under inspection based on detection data of the plurality of first detector units 310 and the plurality of second detector units 320 of the detector assembly 30. According to some embodiments of the present disclosure, the imaging system may use iterative reconstruction algorithms, analytical reconstruction algorithms, or a combination of different reconstruction algorithms when reconstructing the three-dimensional scanning image of the objection under inspection. For example, if the ray source assemblies 10 and 20 have a combined scanning angle greater than 180 degrees relative to the inspection area, the imaging system can prioritize the analytical reconstruction algorithm to improve reconstruction speed and reduce computational performance requirements. For example, if the ray source assemblies 10 and 20 have a combined scanning angle less than 150 degrees relative to the inspection area, the imaging system can prioritize the iterative reconstruction algorithm or use analytical results as initial values of the iterative algorithm. According to some embodiments of the present disclosure, recognition algorithm adopted by the imaging system can be based on either three-dimensional scanning (CT) images or digital radiography (DR) images, or based on both of the CT images and DR images.

According to some embodiments of the present disclosure, each ray source 100 has a scanning angle. In this article, the "scanning angle" of each ray source 100 represents an angle range of all target spots of the corresponding ray source 100 relative to the inspection area (for example, relative to a central axis of the inspection area). In this article, the central axis of the inspection area represents an axis that passes through an approximate center of the inspection area when observed in the direction of travel and is substantially perpendicular to the detector bracket central plane. According to some embodiments of the present disclosure, when the imaging system includes a plurality of ray sources 100, the plurality of ray sources 100 (all the ray sources 100 of the ray source assemblies 10 and 20) located at different scanning positions relative to the inspection area can provide a combined scanning angle. In this article, the "combined scanning angle" refers to a scanning angle generated by combining the scanning angles of the plurality of ray sources 100 at a plurality of scanning positions relative to the inspection area. In some embodiments, the combined scanning angle of the plurality of ray sources 100 located at the plurality of scanning positions relative to the inspection area can be continuous or discontinuous.

According to some embodiments of the present disclosure, the ray source assemblies 10 and 20 have a combined scanning angle greater than 120 degrees relative to the inspection area when observed in the direction of travel. When the combined scanning angle of the imaging system is greater than 120 degrees, the imaging system can basically achieve the three-dimensional image reconstruction. In an exemplary embodiment, when observed in the direction of travel, the ray source assemblies 10 and 20 have a combined scanning angle greater than 180 degrees relative to the inspection area. When the imaging system has a combined scanning angle greater than 180 degrees, it can generate more complete scanning data, and generate better CT scanning effect and better three-dimensional scanning image.

According to some embodiments of the present disclosure, the imaging system is configured such that the X-rays emitted by the ray source assembly 10 can pass through the inspection area and be received by the plurality of first detector units 310, and that the X-rays emitted by the ray source assembly 20 can pass through the inspection area and be received by the plurality of second detector units 320. In some embodiments, the imaging system is configured such that the X-rays emitted by the ray source assembly 10 can cover the first detector units 310 in the direction of travel and/or the X-rays emitted by the ray source assembly 20 can cover the second detector units 320 in the direction of travel.

According to some embodiments of the present disclosure, the imaging system is configured such that the X-rays emitted by each ray source 100 will not be blocked by the detector assembly 30 before passing through the inspection area. In an exemplary embodiment, each first detector unit 310 of the detector assembly 30 is arranged to not block the X-rays emitted by one or more ray sources 100 of the ray source assembly 10 on the same side as the first detector unit 310, and can receive X-rays emitted by one or more ray sources 100 of the ray source assembly 10 on other sides. In an exemplary embodiment, each second detector unit 320 of the detector assembly 30 is arranged to not block the X-rays emitted by one or more ray sources 100 of the ray source assembly 20 on the same side as the second detector unit 320, and can receive X-rays emitted by one or more ray sources 100 of the ray source assembly 20 on other sides.

Below, the arrangement of the ray source and the detector unit according to some embodiments of the present disclosure is described in detail with reference to the accompanying drawings.

According to some embodiments of the present disclosure, the target spots of each ray source 100 of the ray source assembly 10 are arranged to deflect towards the plurality of first detector units 310 in the direction of travel by a first deflection angle, such that the X-rays emitted by each ray source 100 of the ray source assembly 10 can pass through the inspection area and be received by the plurality of first detector units 310. In some embodiments, the first deflection angle is configured such that the X-rays emitted by each ray source 100 of the ray source assembly 10 can cover the first detector units 310 in the direction of travel. In some embodiments, the first deflection angle is configured such that the X-rays emitted by each ray source 100 of the ray source assembly 10 will not be blocked by the plurality of first detector units 310 before passing through the inspection area.

According to some embodiments of the present disclosure, the target spots of each ray source 100 of the ray source assembly 20 are arranged to deflect towards the plurality of second detector units 320 in the direction of travel by a second deflection angle, such that the X-rays emitted by each ray source 100 of the ray source assembly 20 can pass through the inspection area and be received by the plurality of second detector units 320. In some embodiments, the second deflection angle is configured such that the X-rays emitted by each ray source 100 of the ray source assembly 20 can cover the second detector units 320 in the direction of travel. In some embodiments, the second deflection angle is configured such that the X-rays emitted by each ray source 100 of the ray source assembly 20 will not be blocked by the plurality of second detector units 320 before passing through the inspection area.

In an exemplary embodiment, the first deflection angle is equal to the second deflection angle.

Fig. 6A shows a cross-sectional schematic diagram of a ray source and a detector unit according to some embodiments of the present disclosure. In an exemplary embodiment, as shown in Fig. 6A, the ray source 100 is deflected towards the detector units 310 (or 320) in the direction of travel by an angle. Please note that in Fig. 6A, the detector units on the same side as the ray source 100 are not shown but only one detector unit opposite the ray source 100 is shown. In some embodiments, each ray source 100 is deflected around its target spot axis. In an exemplary embodiment, each ray source 100 is deflected such that the X-rays emitted by the ray source 100 can cover the corresponding detector units 310 (or 320) in the direction of travel. By deflecting the ray source 100 relative to the detector units, the X-rays emitted by the ray source 100 can be received by the corresponding detector units more effectively. Further, the X-rays emitted by the ray source 100 can avoid the detector units on the same side as the ray source and can further be received by the detector units on other sides.

According to some embodiments of the present disclosure, the ray source assembly 10 may further include a collimator adapted for deflecting a direction of the X-rays emitted by the ray sources 100 of the first ray source assembly 10 towards the plurality of first detector units 310 in the direction of travel by a first deflection angle, so that the X-rays emitted by the ray sources 100 of the ray source assembly 10 can pass through the inspection area and be received by the plurality of first detector units 310. In some embodiments, the first deflection angle is configured such that the X-rays emitted by each ray source 100 of the ray source assembly 10 can cover the first detector units 310 in the direction of travel. In some embodiments, the first deflection angle is configured such that the X-rays emitted by each ray source 100 of the ray source assembly 10 will not be blocked by the plurality of first detector units 310 before passing through the inspection area.

According to some embodiments of the present disclosure, the ray source assembly 20 may further include a collimator adapted for deflecting a direction of the X-rays emitted by the ray sources 100 of the second ray source assembly 20 towards the plurality of second detector units 320 in the direction of travel by a second deflection angle, so that the X-rays emitted by the ray sources 100 of the ray source assembly 20 can pass through the inspection area and be received by the plurality of second detector units 320. In some embodiments, the second deflection angle is configured such that the X-rays emitted by each ray source 100 of the ray source assembly 20 can cover the second detector units 320 in the direction of travel. In some embodiments, the second deflection angle is configured such that the X-rays emitted by each ray source 100 of the ray source assembly 20 will not be blocked by the plurality of second detector units 320 before passing through the inspection area.

In an exemplary embodiments, the first deflection angle is equal to the second deflection angle.

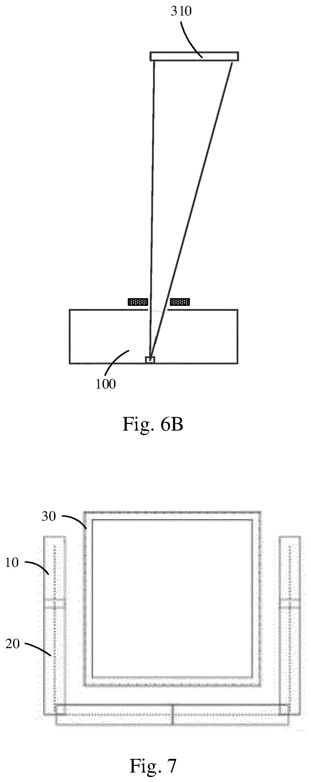

Fig. 6B shows a cross-sectional schematic diagram of a ray source and a detector unit according to some embodiments of the present disclosure. In an exemplary embodiment, as shown in Fig. 6B, the X-rays emitted by the ray source 100 are constrained by the collimator (as shown by shaded portions), so that the direction of the X-rays emitted by the ray source 100 is deflected towards the detector unit 310 (or 320) by an angle. Please note that the detector units on the same side as the ray source 100 is not shown in Fig. 6B, and merely one detector unit opposite the ray source 100 is shown. In an exemplary embodiment, the collimator is configured to deflect the X-rays emitted by the ray source 100 to cover the corresponding detector units 310 (or 320) in the direction of travel. By using the collimator to deflect the direction of the X-rays emitted by the ray source 100, the X-rays emitted by the ray source 100 can be more effectively received by the corresponding detector units. Further, the X-rays emitted by the ray source 100 can avoid the detector units on the same side as the ray source and can further be received by the detector units on other sides.

As described above, by deflecting the ray sources, or by using the collimator, the X-rays can be received by the detector units. However, the present disclosure is not limited to this. According to some embodiments of the present disclosure, the ray sources 100 of the imaging system can further be configured so that the emitted X-rays have a beam width sufficiently wide in the direction of travel to cover the detector units 310 or 320 in the direction of travel. For example, the collimator of the ray source 100 can have a wider slit width. In this case, some of the X-rays emitted by the ray source 100 will fall outside a receiving range of the detector units. In some embodiments, the imaging system may further be provided with additional shielding components (for example, located on one side of the detector units in the direction of travel) to shield the X-rays falling outside the receiving range of the detector units.

In some embodiments, the detector assembly 30 is disposed closer to a center of the inspection area relative to the ray source assemblies 10 and 20. In some embodiments, when the imaging system includes a plurality of ray sources 100, the detector assembly 30 is disposed closer to the center of the inspection area relative to all the ray sources 100. Therefore, the detector assembly 30 is located on an inner side of the ray source assemblies 10 and 20 in a radial direction.

According to some embodiments of the present disclosure, the number of target spots in the ray source assembly 10 may be the same as or different from the number of target spots in the ray source assembly 20. According to some embodiments of the present disclosure, when observed in the direction of travel, distribution positions of the target spots in the ray source assembly 10 do not completely coincide with distribution positions of the target spots in the ray source assembly 20. Fig. 7 is a schematic diagram of target spot distribution of an imaging system according to some embodiments of the present disclosure. In some embodiments, as shown in Fig. 7, the target spot distribution in the ray source assembly 10 and that in the ray source assembly 20 do not completely coincide with each other. Therefore, the distribution position of the target spots in the ray source assembly 10 and that in the ray source assembly 20 can complement each other to increase the number of target spots for effective imaging and provide a larger combined scanning angle.

In some embodiments, when observed in the direction of travel, the distribution positions of the target spots in the ray source assembly 10 are staggered from those of the target spots in the ray source assembly 20. Therefore, the target spots in the ray source assemblies 10 and 20 are distributed to avoid any two target spots from being located at the same position. Therefore, the imaging system can fully utilize the target spots respectively in the ray source assemblies 10 and 20 which are completely staggered from each other for imaging, and can improve imaging accuracy and quality.

According to some embodiments of the present disclosure, when observed in the direction of travel, a projection of each ray source 100 of the ray source assembly 10 does not completely fall within a projection of any ray source 100 of the ray source assembly 20, and the projection of each ray source 100 of the ray source assembly 20 does not completely fall within the projection of any ray source 100 of the ray source assembly 10. In this case, for example, as shown in Figs. 1 and 2, the arrangements of the respective ray sources 100 in the ray source assembly 10 and in the ray source assembly 20 are not completely consistent, and there is no ray source 100 in the ray source assemblies 10 and 20 which completely coincides with any of other ray sources 100 or completely falls within a range of another ray source 100. As such, the ray emission range and scanning range of the ray source assembly 10 and that of the ray source assembly 20 can complement each other, thereby increasing the number of target spots for effective imaging and providing a larger combined scanning angle.

In some embodiments, when observed in the direction of travel, the projections of the plurality of ray sources 100 of the ray source assembly 10 are staggered from those of the plurality of ray sources 100 of the ray source assembly 20. In this case, the respective ray sources 100 in the ray source assembly 10 and the ray source assembly 20 are completely staggered with each other. As a result, the imaging system can fully utilize all the ray sources of the ray source assemblies 10 and 20 for imaging, and can improve imaging accuracy and quality.

According to some embodiments of the present disclosure, the imaging system may further include a ray source control device. The ray source control device is adapted to control ray emission of the ray source assembly 10 and the ray source assembly 20. In some embodiments, the ray source control device is configured so that at most one target spot in the ray source assembly 10 emits X-rays and at most one target spot in the ray source assembly 20 emits X-rays at the same moment. Therefore, there is no more than two target spots emitting X-rays simultaneously in the first ray source plane, and there is no more than two target spots emitting X-rays simultaneously in the second ray source plane. At the same moment, the imaging system can have one target spot from the first ray source plane and one target spot from the second ray source plane emitting X-rays simultaneously. According to some embodiments of the present disclosure, the ray source control device can be configured to control the ray emission of the ray source assembly 10 and that of the ray source assembly 20, respectively, such as emission sequence, emission frequency, emission current of the target spots, and the like.

According to some embodiments of the present disclosure, at least one target spot in the ray source assemblies 10 and 20 is a digital radiographic (DR) target spot. After being received by the detector assembly 30, the X-rays emitted by the DR target spot can be adapted to generate DR images. Therefore, the imaging system according to some embodiments of the present disclosure can be used for both CT imaging and DR imaging. In some embodiments, the ray source control device is configured so that the ray emission frequency of the digital radiographic target spot is higher than that of other target spots. In some embodiments, the imaging system includes multiple DR target spots selected from the ray source assemblies 10 and 20. In some embodiments, when the imaging system includes multiple DR target spots, these DR target spots can be located at different scanning positions relative to the inspection area, that is, being adapted to generate DR images from different angles of view.

As described above, one or more target spots of the ray source assemblies 10 and 20 are selected as DR target spots. However, the present disclosure is not limited to this. In some embodiments, the imaging system can further be provided with separate DR target spots, that is, the DR target spots are independent of the target spots in the ray source assemblies 10 and 20. In this case, the imaging system can further be provided with a separate DR detector unit to receive X-rays emitted by the separate DR target spots.

It should be understood that the arrangement, quantity, and shape of the ray source and the detector unit mentioned above are only illustrative and should not be considered as limiting the present disclosure.

Below, an imaging method according to some embodiments of the present disclosure is described in detail. According to some embodiments of the present disclosure, the imaging method can be implemented by any of the above imaging systems.

Below, the imaging method according to some embodiments of the present disclosure is described by taking an object under inspection passing across the ray source assembly 10 and the ray source assembly 20 in sequence, as an example. However, it should be understood that the object under inspection can pass across the ray source assembly 20 and the ray source assembly 10 in sequence.

In step S10, the object under inspection is carried on the conveying device and the conveying device transports the object under inspection through the inspection area in the direction of travel. In step S20, the target spots of the ray source assemblies 10, 20 are controlled to emit X-rays in a predetermined sequence. In step S30, the emitted X-rays penetrate the object under inspection located in the inspection area and are received by the detector assembly 30.

In some embodiments, the imaging method may further include a step S40: reconstructing a three-dimensional scanning image of the object under inspection based on detection data from the plurality of first detector units 310 and the plurality of second detector units 320. In some embodiments, the imaging method may further include recognizing the object under inspection and providing recognition results after reconstructing the three-dimensional scanning image of the object under inspection. In some embodiments, the imaging method may further include displaying the three-dimensional scanning image and/or the recognition results.

In some embodiments, the imaging method may further preload or generate configuration information, correction information, or the like, such as background data and air data, before performing the step S10.

In some embodiments, the imaging method may further include detecting whether the object under inspection has entered beam surfaces of the X-rays emitted by the ray source assembly 10 through the plurality of first detector units 310 of the detector assembly 30. In some embodiments, the imaging method may further include detecting whether the object under inspection has entered beam surfaces of the X-rays emitted by the ray source assembly 20 through the plurality of second detector units 320 of the detector assembly 30. For example, by detecting in real-time whether the object under inspection has entered the beam surfaces of the X-rays of the ray source assemblies 10 or 20, the imaging method can provide reference for subsequent operations.

In some embodiments, the imaging method may further include caching and/or preprocessing detection data of the plurality of first detector units 310 when it is determined that the object under inspection has entered the beam surfaces of the X-rays emitted by the ray source assembly 10. In some embodiments, when it is determined that the object under inspection has entered the beam surfaces of the X-rays emitted by the ray source assembly 20, the imaging method can begin to reconstruct the three-dimensional scanning image of the object under inspection based on the detection data of the plurality of first detector units 310 and the plurality of second detector units 320. In some embodiments, the imaging method may further include controlling the ray source assemblies 10, 20 to stop emitting the X-rays when it is determined that the object under inspection has completely passed through the beam surfaces of the X-rays emitted by the ray source assembly 20.

In some embodiments, the imaging method may further include controlling the DR target spot to emit X-rays so as to generate DR images. In some embodiments, when a separate DR target spot is provided, the imaging method can receive X-rays that are emitted by the separate DR target spot and pass through the object under inspection through a separate DR detector unit. In some embodiments, when one or more target spots in the ray source assemblies 10 and 20 are selected as DR target spots, the imaging method can receive X-rays that are emitted by the DR target spots and pass through the object under inspection through the detector assembly 30.

In the imaging methods according to some embodiments of the present disclosure, other embodiments of the used imaging system used are described above and correspondingly incorporated into the embodiments of the imaging method, and will not be repeated here.

Although the present disclosure has been described with reference to exemplary embodiments, it should be understood that the present disclosure is not limited to the configurations and methods of the embodiments as mentioned above. On the contrary, the present disclosure is intended to cover various modifications and equivalent configurations. In addition, although various disclosed elements and method steps are shown in various exemplary combinations and configurations, other combinations including more or fewer elements or methods also fall within the scope of the present disclosure.

## Claims

1. An imaging system for radiographic inspection, comprising:
an inspection area, wherein an object under inspection can be transported through the inspection area in a direction of travel;
a first ray source assembly, comprising a plurality of ray sources;
a second ray source assembly, comprising a plurality of ray sources, wherein each ray source of the first ray source assembly and of the second ray source assembly comprises a separate shell to define a vacuum space and comprises a plurality of target spots enclosed in the shell, the target spots of all the ray sources of the first ray source assembly are arranged in a first ray source plane, and the target spots of all the ray sources of the second ray source assembly are arranged in a second ray source plane;
a plurality of first detector units, adapted for receiving X-rays that are emitted from the first ray source assembly and pass through the inspection area, the plurality of first detector units being arranged in a first detector plane;
a plurality of second detector units, adapted for receiving X-rays that are emitted from the second ray source assembly and pass through the inspection area, the plurality of second detector units being arranged in a second detector plane; and
a detector bracket, wherein the plurality of first detector units and the plurality of second detector units are installed on the detector bracket,
wherein the first ray source plane, the first detector plane, the second detector plane, and the second ray source plane are distributed in sequence in the direction of travel.

2. The imaging system according to claim 1, wherein the first ray source plane, the first detector plane, the second detector plane, and the second ray source plane are substantially perpendicular to the direction of travel and parallel to each other.

3. The imaging system according to claim 1 or 2, wherein the imaging system is further configured to reconstruct a three-dimensional scanning image of the object under inspection based on detection data from the plurality of first detector units and the plurality of second detector units.

4. The imaging system according to claim 3, further comprising a ray source control device for controlling ray emission of the first ray source assembly and the second ray source assembly, wherein the ray source control device is configured so that at the same moment, at most one target spot in the first ray source assembly emits X-rays and at most one target spot in the second ray source assembly emits X-rays.

5. The imaging system according to claim 1 or 2, wherein the plurality of first detector units cover at least a ray emission range of the first ray source assembly, and the plurality of second detector units cover at least a ray emission range of the second ray source assembly.

6. The imaging system according to claim 5, wherein the plurality of first detector units are configured to extend completely around the inspection area to form a first detector ring; and/or
the plurality of second detector units are configured to extend completely around the inspection area to form a second detector ring.

7. The imaging system according to claim 1 or 2, wherein when observed in the direction of travel, distribution positions of the target spots in the first ray source assembly do not completely coincide with distribution positions of the target spots in the second ray source assembly.

8. The imaging system according to claim 7, wherein when observed in the direction of travel, the distribution positions of the target spots in the first ray source assembly are staggered from those of the target spots in the second ray source assembly.

9. The imaging system according to claim 1 or 2, wherein when observed in the direction of travel, a projection of each ray source of the first ray source assembly does not completely fall within a projection of any ray source of the second ray source assembly, and the projection of each ray source of the second ray source assembly does not completely fall within the projection of any ray source of the first ray source assembly.

10. The imaging system according to claim 9, wherein when observed in the direction of travel, projections of the plurality of ray sources of the first ray source assembly are staggered from those of the plurality of ray sources of the second ray source assembly.

11. The imaging system according to claim 1 or 2, wherein,
all the target spots of each ray source of the first ray source assembly are arranged to deflect towards the plurality of first detector units in the direction of travel by a first deflection angle, so that the X-rays emitted by each ray source of the first ray source assembly will not be blocked by the plurality of first detector units before passing through the inspection area; and/or
all the target spots of each ray source of the second ray source assembly are arranged to deflect towards the plurality of second detector units in the direction of travel by a second deflection angle, so that the X-rays emitted by each ray source of the second ray source assembly will not be blocked by the plurality of second detector units before passing through the inspection area.

12. The imaging system according to claim 1 or 2, wherein,
the first ray source assembly further comprises a first collimator adapted for deflecting a direction of the X-rays emitted by the first ray source assembly towards the plurality of first detector units in the direction of travel by a first deflection angle; and/or
the second ray source assembly further comprises a second collimator adapted for deflecting a direction of the X-rays emitted by the second ray source assembly towards the plurality of second detector units in the direction of travel by a second deflection angle.

13. The imaging system according to claim 1 or 2, wherein when observed in the direction of travel, the first ray source assembly and the second ray source assembly have a combined scanning angle greater than 120 degrees relative to the inspection area.

14. The imaging system according to claim 13, wherein when observed in the direction of travel, the first ray source assembly and the second ray source assembly have a combined scanning angle greater than 180 degrees relative to the inspection area.

15. The imaging system according to claim 4, wherein at least one target spot in the first ray source assembly and the second ray source assembly is a digital radiographic "DR" target spot, and the ray source control device is configured so that a ray emission frequency of the digital radiographic target spot is higher than that of other target spots.

16. The imaging system according to claim 1 or 2, wherein the first detector unit comprises a single row of detector crystals or multiple rows of detector crystals, and/or the second detector unit comprises a single row of detector crystals or multiple rows of detector crystals.
